# EUROPEAN PATENT APPLICATION

(11) **EP 4 704 035 A1**
(43) Date of publication of application: **04.03.2026**
(21) Application number: 25197802.9
(22) Date of filing: 25.08.2025
(51) Int. Cl.: G06T 7/73, A61B 8/00

(54) **MARKERLESS POSE ESTIMATION OF A MEDICAL DEVICE FROM A SINGLE CAMERA**

(30) Priority: 27.08.2024 US 202418816357
(71) Applicant: Siemens Medical Solutions USA, Inc., Malvern, PA 19355 (US)
(72) Inventor: SHENOY, Nikhil, West Windsor, NJ 08550 (US); KIM, Young-Ho, West Windsor, NJ 08550 (US); KAPOOR, Ankur, Plainsboro, NJ 08536 (US)
(74) Representative: HKW Intellectual Property PartG mbB

(57) **Abstract**

The pose of medical device in use, where the device may be occluded, is estimated using a single camera. Multiple cameras and/or a marker are not needed. The medical device is detected in the camera image during use to determine the pose. Various further approaches may be used to deal with occlusion. Comparison with a template for that type of medical device may be used to deal with occlusion. Modeling the occlusion, such as the hand, may be used to deal with occlusion.

## Description

### BACKGROUND

The present embodiments relate to pose estimation for a medical device, such as an ultrasound transducer. Medical device tracking is a key component of modern scanning and/or surgical procedures. Tracking the device synchronizes the location of the device with respect to the anatomy, reducing the chance of errors.

Stereo-based optical tracking may be used. Two or more cameras observe the operating scene. To identify the device in the stereo view, specially designed markers (e.g., infrared reflectors or light emitting diodes) are fitted onto the body of the device, such as in a removable housing. Adding markers or acquiring devices with pre-designed markers may be expensive. Markers may be occluded by the user, such as a hand or arm intervening between the marker and one or both of the cameras.

Electromagnetic tracking uses Faraday's Law to detect movement of the device, but a receiver module has to be added to the device being tracked. The device may be tracked even when occluded, but the tracking is not as precise as in an optical solution.

Optical tracking without a marker has been proposed. No prior information is used for tracking. Common objects, such as a pitcher or drill, are tracked by segmenting a moving object from a video stream. Pose is based on extracted features using graph-based alignment. While this approach may work on general purpose objects, rare shapes found in a medical environment and occlusion may result in erroneous tracking, particularly where substantial rotation of the device occurs.

In another general approach, the pose of a previously unseen object, such as used in robotics, is estimated. Prior object information in the form of a CAD model or other 3D object representation is used in conjunction with the image properties to render a view of the model into the scene. The rendered object is then compared to another detected object to see whether there is a sufficiently close match. Based on the match, the pose of the object can be estimated. This method may not deal with occlusion well.

### SUMMARY

Systems, methods, and non-transitory computer readable media with stored instructions (program code) are provided for pose estimation. The pose of medical device in use, where the device may be occluded, is estimated using a single camera. Multiple cameras and/or a marker are not needed. The medical device is detected in the camera image during use to determine the pose. Various further approaches may be used to deal with occlusion. Comparison with a template for that type of medical device may be used to deal with occlusion. Modeling the occlusion, such as the hand, may be used to deal with occlusion.

In a first aspect, a method is provided for pose estimation of an ultrasound transducer. A single camera captures an image of the ultrasound transducer. The ultrasound transducer as captured in the image is being held and occluded by a user. The ultrasound transducer is detected in the image. A pose of the ultrasound transducer as detected in the image from the single camera is determined. Ultrasound imaging using the ultrasound transducer has scan data aligned by the determined pose.

In a second aspect, an ultrasound system is provided. A single camera is configured to image an acoustic transducer occluded in part by a user. An image processor is configured to determine a pose of the acoustic transducer from an image from the single camera. The determination is based on a template of the transducer. The image processor may be implemented to carry out a method according to the first aspect.

In a third aspect, a method is provided for pose estimation of a medical device. A single camera captures an image of the medical device during use of the medical device on or in a patient. The medical device is held and occluded by a user in the image. The medical device is detected in the image, and a part of the user is detected in the image. A pose of the medical device as detected in the image from the single camera is determined utilizing the detected part of the user in the image. The pose estimation for the medical may be implemented according to the first aspect wherein the ultrasound transducer is replaced by a general medical device.

Any one or more of the aspects or concepts summarized above or in the Illustrative Embodiments below may be used alone or in combination. The aspects or concepts described for one Illustrative Embodiment or aspect may be used in other embodiments or aspects. The aspects or concepts described for a method or system may be used in others of a system, method, or non-transitory computer readable storage medium.

The present invention is defined by the following claims, and nothing in this section should be taken as a limitation on those claims. Further aspects and advantages of the invention are discussed below in conjunction with the preferred embodiments and may be later claimed independently or in combination.

### BRIEF DESCRIPTION OF THE DRAWINGS

The components and the figures are not necessarily to scale, emphasis instead being placed upon illustrating the principles of the embodiments. Moreover, in the figures, like reference numerals designate corresponding parts throughout the different views.
Figure 1 is a flow chart diagram of one embodiment of a method for pose estimation of a medical device;
Figure 2 illustrates an example ultrasound transducer;
Figure 3 illustrates the example ultrasound transducer of Figure 2 occluded by fingers or hand of a user; and
Figure 4 is a block diagram of one embodiment of a system for pose determination of a medical device.

### DETAILED DESCRIPTION OF EMBODIMENTS

A single camera with no markers on the imaged object is used for pose estimation, avoiding the costs and complications with extra cameras and markers. In one implementation, a color depth camera (e.g., RGBD camera) observes the target medical object in the medical environment. A representation of the target object is formed from the image (e.g., RGBD data). The object itself is not augmented with any additional identifying markers; objects are identified as they appear and are used in the operating environment. The approach is for a set of known objects limited to the set of tools typically used for a medical procedure. Once the object is identified in the scene, key features are extracted from the observed object. These features represent important characteristics of the object that can be used for matching against a ground truth template. Since the pose of a set of known objects is to be detected, the prior ground truth geometrical information about the object is known. This can be in the form of CAD files or another object representation. Key features are extracted from the ground truth object representation and matched against the key features from the detected object. Once the matching is completed, then the pose of the object relative to the camera is calculated.

A single camera performs the object tracking without an added marker. Using a single camera reduces the amount of physical hardware to construct the tracking setup and also reduces the need for complicated calibration techniques to calibrate multiple cameras. The less stringent hardware requirement brings down the overall cost of the system. Markerless detection removes the burden of attaching specialized tracking hardware to all the tools that an operator may need during a procedure. Adding markers adds time to the procedure and can create errors if the markers are not attached correctly. The markerless system allows the operator to use the tools as they are. Only a one-time loading of the object library (templates) allows the single camera to determine pose, which one-time loading may be performed during the manufacturing of the system or in a software update.

Occlusion is managed robustly by considering the shape geometry of the medical device along with any other objects that may occlude the medical device. In the case of ultrasound scanning, a hand will most likely occlude a significant portion of the probe body. The hand and its interaction with the probe are modeled to accurately determine the pose. Occlusions could happen by the hand, one or more other objects, or from multiple objects. These objects may be modeled.

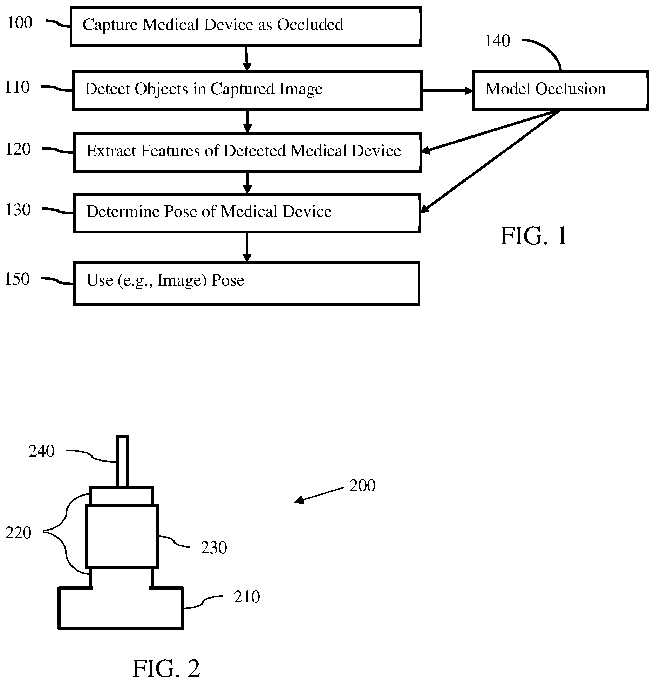

Figure 1 is a flow chart diagram of one embodiment of a method for pose estimation of a medical device, such as an ultrasound transducer. Landmark detection is used for determining the pose as captured by a single camera. Template matching and/or modeling an occluding object may be included. The segmentation of the object may be used to extract features from the mask (segmentation).

The pose of any medical device may be determined. For example, the pose is determined for a surgical instrument, endoscope, or other medical tool held by a physician or nurse. In the examples herein, the medical device is an ultrasound transducer, such as a handheld probe for use external or internally to the patient. The user holds a handle of the ultrasound transducer, and the camera captures an image of the handle and/or another portion of the ultrasound transducer.

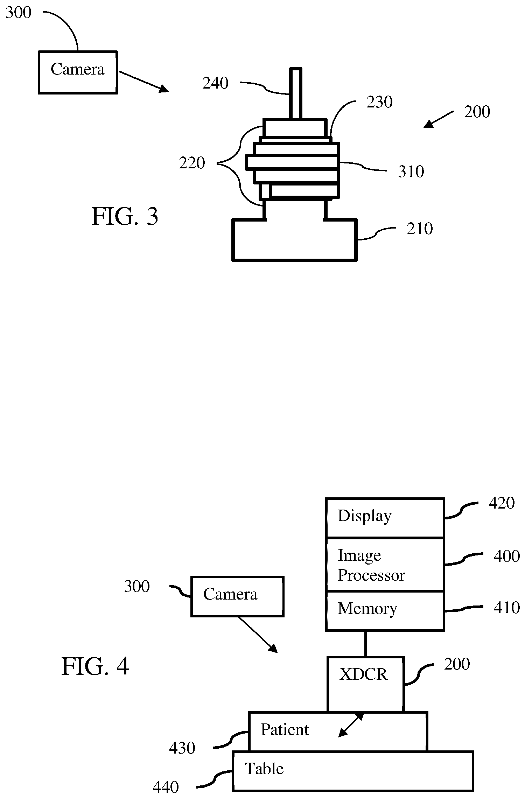

The method of Figure 1 is performed by a medical imaging system, such as an image processor, camera, and/or scanner. A camera captures the scene. An image processor determines the pose from the captured scene. A memory may store one or more templates. The image processor may be part of a medical scanner, such as an ultrasound system, or may be a separate computer, workstation, or processor. The image processor, medical scanner, physician, or procedure may use the determined pose. In one embodiment, the system of Figure 4 performs the method, but other systems may be used.

Additional, different, or fewer acts may be provided. For example, acts 110 and 120 are combined or performed as one act. As another example, acts 130, 140, and/or 150 are not performed. In another example, acts for configuring a scanner to scan or other use of the medical device are provided.

The acts are performed in the order shown (top to bottom or numerical) or another order. For example, act 140 may be performed as part of act 110, 120, 130, or separately before or after act 120.

In act 100, a single camera captures an image of the medical device (e.g., ultrasound transducer). The medical device is being held and occluded by a user or arm (e.g., robotic arm) in the image. For example, Figures 2 shows a handheld transducer 200 while not being held. Figure 3 shows the handheld transducer 200 with at least part of the transducer 200 occluded by the hand 310 (e.g., fingers) of the user. The image captured by the camera is of the ultrasound transducer 200 during use (e.g., Figure 3) of the ultrasound transducer 200 on or in the patient. The ultrasound transducer 200 is being held or moved by the user to scan the patient. The hand, fingers, palm, arm, body, head, and/or another part of the user occludes part of the ultrasound transducer 200 relative to the camera 300. As a result, part of the ultrasound transducer 200 as captured in the image is occluded by the user.

The camera captures an image of the scene in the field of view of the camera. The camera is positioned to capture the ultrasound transducer in the scene while being used on or for the patient. One or more images are captured, such as a still shot or a video.

The camera captures the image as a two-dimensional distribution of pixels, such acquiring red, green, blue (RGB) values. Other information, such as depth information, may be captured as or as part of the image. Thermal, infrared, or another image may be captured.

In one embodiment, the camera is a depth camera, such as a 2.5D or RGBD (RGB plus depth) camera (e.g., Microsoft Kinect 2 or ASUS Xtion Pro). One sensor may capture color image and another sensor of the depth camera captures depth. The depth camera may directly measure depths, such as using time-of-flight, interferometry, or coded aperture. The depth camera (e.g., RGBD camera) outputs a color point cloud where each pixel has a color and depth. Other object representations may be used. While constructing the point cloud is one method, the color and depth images may be used directly, avoiding reconstructing a point cloud. Other optical or non-ionizing sensors may be used, such as a LIDAR camera.

Only one camera is used. For example, the single depth camera is used. Where the depth camera uses separate color and depth sensors in the same camera device, these separate data are combined to generate the representative point cloud or representation of the target device.

The camera may be placed in various locations in the room, including attached or connected to a medical scanner, wall, ceiling, or headset. The camera is positioned on a wall, ceiling, or elsewhere in the imaging suite or operating room, such as on a boom generally above the patient.

The camera is directed at a patient and/or region where the ultrasound transducer is to be used on or in the patient. The camera's field of view covers the region where the ultrasound transducer is to be used. The camera captures the outer surface of the ultrasound transducer, patient, and/or user from one perspective. The camera is mounted to limit or avoid occlusion and/or to maximize visualization of features used to determine the pose.

The image is used at the resolution of the sensor. For example, the image or point cloud is at 256x256 pixels. Other sizes may be used, including rectangular fields of view. The image may be filtered and/or processed. For example, the image is altered to a given resolution. As another example, the image is down sampled, such as reducing 256x256 to 64x64 pixels. The image may be cropped, such as limiting the field of view.

The image is captured for a given time. Using continuous or defined frequency of capture, a stream of images (i.e., video) from different times may be captured. The defined frequency may be preset, adjustable, or variable. By capturing images over time, images form a video of the ultrasound transducer during use on or in the patient. In alternative implementations, an image for just one time is captured.

The image shows the ultrasound transducer free of any added optical marker. A target, color code, or shape other than the shape of the probe itself is not added. The ultrasound transducer itself is not augmented with any additional identifying markers for pose determination. Trademark or brand markers may be included, but no markers added for pose determination. The ultrasound transducer is to be identified as the ultrasound transducer appears or is shipped from the manufacturer and is used in the operating environment. Since triangulation for stereo viewing is not being used, added markers are not needed. A marker may be added for other purposes or left over from use with other pose determination approaches, but the marker is not needed.

In act 110, the image processor detects the ultrasound transducer in the image. For example, the parts or pixels of the color point cloud belonging to or representing the ultrasound transducer are detected. The ultrasound transducer is segmented in or from the image. The locations or representation of the ultrasound transducer is distinguished from other objects in the image. Pixels only belonging to the ultrasound transducer are isolated or labeled.

Any segmentation may be used. For example, a random walker, threshold, region growing, or other hand-coded segmentation is applied. As another example, a machine-learned classifier, such as a deep-learned neural network or another machine-learned model, segments (outputs segmentation in response to input of the image). The machine-learned classifier may have been trained using example images or videos captured during scans of the patient with ground truth determined manually on the images or videos or with another process. Synthetic data may be used for training examples. During the model training, a CAD model along with RGBD data of transducers collected beforehand in the real world are collected. In contrast to this data, simulated RGBD data for training is created by importing the CAD model into a simulation environment and generating synthetic scenes where the transducer is scanning a patient. This allows generation of a large amount of synthetic data with a minimal amount of effort. The model machine training process may use real-world and/or synthetic data to learn the object shape.

Other objects may be segmented. For example, the user or part of the user (e.g., hand) is segmented. The user as represented in the image is detected, and locations belonging to the user are distinguished from other locations. The same or different algorithm or machine-learned classifier may be used to segment different objects, such as objects occluding the ultrasound transducer. Other medical devices being used in a procedure, such as surgical tools, may be detected as well as the ultrasound transducer. Any number of segmentations for a given image may be performed, such as segmenting for any medical device in a set of possible medical devices.

Some filtering and/or smoothing may remove noisy data prior to or after segmentation. The segmentation generates a representation of the ultrasound transducer with or without generating representations of other objects.

Three-dimensional reconstruction may be used. The camera properties or parameters are used to reconstruction a three-dimensional object or surface for the ultrasound transducer and/or for other objects (e.g., hand). Detection by segmentation and 3D reconstruction generates a representation of the ultrasound transducer from the camera viewpoint for further analysis. Similarly, a representation of the hand or other occluding object from the camera viewpoint is generated. Part of the user (e.g., occluding part) is detected in the image. The segmentation may be used without further three-dimensional reconstruction. Using a depth camera, the segmentation represents the object as a three-dimensional surface.

In act 120, the image processor extracts a plurality of features of the detected ultrasound transducer and/or other objects in the image. Landmarks are detected. For example, key points or landmarks of the ultrasound transducer are detected from the segmented representation of the ultrasound transducer. Once the ultrasound transducer is identified in the scene, key features are extracted from the observed object. These features represent important characteristics of the object that can be used for matching against a ground truth template.

The extraction of features may be by processing, such as template matching or edge detection, or may be by application of a machine-learned model. For a machine-learned model, the segmented or detected representation is input to the model. Alternatively, or additionally, information derived from the segmented representation is input. The machine-learned model is trained to output one or more landmarks in response to the input. A neural network, support vector machine, or Bayesian inference model may be used.

Any landmarks may be extracted as features. The extraction detects the landmark position in the representation. The extraction may be of semantic features, such as using a semantic feature extractor. Semantic feature extractors discover features that a human would inherently understand are important about an object. For example, the corners of a cube and the centers of each face of the cube could be considered semantic features. Figure 2 shows an example ultrasound transducer 200. The transducer 200 includes an elongated region 210 housing an array and a handheld region 220 with a grip 230. A cable 240 connects to the handheld region 220. Example semantic features may include edges of the grip 230, corners and/or edges of the region 210 and/or region 220. The contact point of the cable 240 with the region 220 may be another semantic feature. Additional, different, or fewer semantic features may be provided.

The extraction may be of general features or learned features, such as using a general feature extractor. In one approach, the general feature extractor is a neural network, such as an encoder, that outputs a feature vector based on machine training. The feature vector is an abstraction created by deep learning. General feature extractors may capture semantic features but can also capture features that are not so obvious (e.g., general cues). This can include features generated based on lighting or surface texture, as well as subtle cues about the geometry of the object. The detected features are invariant to the viewpoint from which the camera is observing the object. Either or both types of feature extractor can be used. Other feature extractors or landmark detection may be used. The same feature extractor or class of feature extractor is applied to the observed or detected ultrasound transducer and a ground truth template.

In other approaches, the image processor extracts the features as part of detection. Acts 110 and 120 are combined.

Some features may not be extracted. For example, the user occludes one or more features. In the example of Figure 3, the hand 310 of the user occludes any features, semantic or general, associated with most of the grip 230. Similarly, any features on the opposite side of the ultrasound transduce 200 from the camera 300 are not shown in the image so are not extracted.

In act 130, the image processor determines a pose of the ultrasound transducer. The image processor determines the position, orientation, scale, and/or combinations of two or more thereof of the ultrasound transducer relative to the camera.

The segmented ultrasound transducer provides a point cloud or spatial positions of the ultrasound transducer relative to the camera. The extracted features, at locations based on the image, provides spatial positions of the ultrasound transducer relative to the camera. The ultrasound transducer as detected in the image from the single camera is located relative to the camera.

Due to occlusion, there may be error introduced in just relying on the segmented object or extracted features for the pose. For more accurate pose determination, the image processor compares the ultrasound transducer as detected (e.g., segmentation and/or extracted features) to an ultrasound transducer template. Different relative poses of the template are compared to the ultrasound transducer as detected. The pose that best matches is identified as the pose of the detected ultrasound transducer. Since the pose of a known type or known object is being determined, prior ground truth geometrical information about the object may be used. A set of known objects and corresponding templates may be provided. The set of objects is limited to the set of tools used for typical list of procedures. Where the specific ultrasound transducer being used is not known, different templates corresponding to different types of transducers may be tested to find the one with the best correlation (e.g., least difference in the best orientation or minimization of the square of the differences). A category-based model of the transducer may be used. For example, a set of ten radial transducers that represent a sampling of the possible variations in transducer shape is provided. The model understands the possible shape variations and can accurately estimate the pose of a radial transducer that is not identical to one of the ten transducers in the learning set because the model knows what a radial transducer looks like in general. A "foundation model" is provided, as the model contains the foundational knowledge of a transducer and its possible shape variations.

The template or templates are computer assisted drawing (CAD) files, a list of spatially related features, encoded feature vectors, or another object representation. The object of the template may be altered in pose for comparison. Alternatively, different templates are provided for different poses.

In one implementation, key features (e.g., semantic and/or generalized) are extracted from the ground truth object representation and matched against the key features from the detected ultrasound transducer. Once the matching is completed, then the pose of the object relative to the camera can be calculated or is determined. The image processor tests different orientations of the ultrasound transducer template to the ultrasound transducer as detected. The testing is a comparison of the segmentation and/or extracted features.

In another implementation, feature vectors output by a machine-learned network (e.g., an encoder) are compared. The ultrasound transducer as detected and/or extracted features are input to the machine-learned network, which outputs a feature vector in response to the input. The template and/or extracted features at a pose to be tested is input to the machine-learned network, which outputs a feature vector in response to the input. Alternatively, a table of pre-processed feature vectors are the template. The feature vectors from the detected ultrasound transducer and the template are compared. Different poses are tested to identify the pose with the best match in the feature vectors.

Where a match occurs, the pose is determined. The testing of different poses results in the pose where a difference in the testing is minimized. Based on calibration, the pose relative to the camera is transformed to pose relative to the patient, ultrasound scanner, global or room coordinates, or another coordinate system.

The template may be specific to the ultrasound transducer being detected, such as the same model or type. In another implementation, the template is generalized or standard to represent a range of different types or models of ultrasound transducers. Different templates may be provided for different styles or generic transducer types, such as a template for different sizes of transducers, different array arrangements, different purposes (e.g., transesophageal verses abdomen scanning), and/or other differences. The comparison for determining pose uses the generalized template or transducer nonspecific to the ultrasound transducer being detected. Sufficient similarities in the extracted features allow for pose determination.

Since the pose is being determined for a generally known object, the feature extractor of act 120 generates a large number (e.g., tens, hundreds, or thousands) of salient features on the ground truth object template. This large number provides a sufficient number of features to match against. The detected ultrasound transducer, due to occlusion and view from a single camera, may lead to less than half or a factor of 10 or fewer features than the template. When viewing the target object in a real-world scenario, it is not possible to view all aspects from the single camera. The features extracted from the view of the target object is a subset of the overall ground truth features. The goal of the matching process is to match this subset to the correct features in the template.

In one implementation, an outlier rejection is used for the comparison. Gaussian filtering, principal component analysis, RANSAC, or another outlier rejection matches the large number of extracted features for the template with the fewer number of extracted features for the detected ultrasound transducer. The minimization to determine the pose also selects the extracted features for the template to use in the comparison. Having many features in both sets to perform the matching helps generate a more accurate solution. The outlier rejection method, such as RANSAC, is used by the image processor to compute the transformation between the target and ground truth to estimate the pose.

In act 140, the image processor models an occluding object or objects. The object may be another medical device, a bed, the patient, a robotic arm, a support arm, a sterile barrier, or another object in the medical environment. For example, a hand of the user is modeled. As shown in Figure 3, the hand 310 of the user occludes part of the ultrasound transducer 200 while in use.

Handling occlusion of the target object (e.g., ultrasound transducer) is a significant problem in pose estimation. Due to occlusion, the pose is determined without the full information of the object. In the case of medical devices and tools, partial to severe occlusion is pervasive in many procedures. Occlusion may occur due the object being partially inserted into the patient or from occlusion of the object's body due to the operator's hand. The second scenario is very common in scanning procedures such as ultrasound, and a camera observing such a scan may not get an unobstructed view of the probe due to the operator's hand blocking the object. This kind of occlusion is accounted for in pose estimation by utilizing information about the occlusion (e.g., hand) of the probe. Since a known object (e.g., ultrasound transducer) is being detected while occluded by a known generic shape (e.g., hand), information from both may be utilized to correctly estimate the pose of the object.

The model or information may be a template, a statistical shape model, a physics-based model, or a biomechanical model. The model is fit to the occluding object. Any fitting may be used, such as performing the detection of act 110, extraction of features of act 120, and determination of pose 130 for the occluding object. The occluding object is detected, and the model is fit to the detected object. An act of scaling using a rigid or affine transformation may be provided.

The fit may indicate a pose of the occluding object, the locations of the ultrasound transducer being occluded, and/or a grip style or placement of the occluding object in interacting with the ultrasound transducer. This information may be used in determining the pose of the ultrasound transducer. The modeling of the occluding object may be used in the detection of the ultrasound transducer (i.e., act 110), the extraction of features of the ultrasound transducer (i.e., act 120), and/or the determination of the pose of the ultrasound transducer (i.e., act 130). By incorporating the model of the occluding object in any of acts 110, 120, and/or 130, the detected occluding object is utilized in the determination of the pose of the ultrasound transducer. In the example of Figure 3, the detected part of the user (i.e., the hand) from the image is utilized in the pose determination.

In one implementation, the image processor performs the detection of act 110 with separate or joint classification of both objects. Joint classification may better distinguish the ultrasound transducer from the hand, improving accuracy of the segmentation and eventual pose determination.

In another implementation, any features extracted in act 120 that may belong to the hand rather than the ultrasound transducer are removed. For example, any features at a hand location are removed even where also identified as a feature of the ultrasound transducer.

In yet another implementation, the orientation, grip style, or other information from the modeled hand is used to indicate the pose of the ultrasound transducer in act 130. For example, the pose of the hand 310 gripping as shown in Figure 3 indicates a pose of the ultrasound transducer 200. The hand pose may be used to initialize the pose search or determination for the ultrasound transducer. The determined pose of the transducer may be confirmed by comparison to the pose of the hand. The poses of the ultrasound transducer and the hand may be averaged. As another example, the solving for the pose of the ultrasound transducer may consider both the ultrasound transducer in the image and the part of the user in the image. The minimization is based on an error or difference in fitting or matching both the ultrasound transducer and the hand. Other utilizations of the modeling of an occluding object may be used.

In act 150, the image processor uses the pose as determined. The pose may be used to position a graphical representation of the ultrasound transducer (or another medical device) relative to pre-operative imaging to assist the physician in guiding the device relative to the patient. The pose may be used to adjust position relative to the patient for scanning, treatment, biopsy, or diagnosis. The pose may be used by a robot or a user (e.g., physician or sonographer). Other uses in the medical environment of the determined pose may be provided.

In one implementation, the pose is used for ultrasound imaging. A one-dimensional array scans an area in the patient. By moving the ultrasound transducer, different areas may be scanned to scan a volume. The pose is used to determine the alignment of each area (i.e., alignment of the ultrasound transducer during each two-dimensional scan) to assemble the volume representation. The scan data is aligned by the determined pose. A three-dimensional representation is rendered from the data of the three-dimensional representation as aligned based on the pose. The alignment may instead, or additionally, be used to show location of scanning relative to the patient and/or pre-operative imaging.

Acts 100-150 may be performed once for an image. In other approaches, act 100 captures a video or sequence of images. Acts 110, 120, and 130 are performed repetitively over time, such as for each image of the sequence.

The repetitions determine the pose independent of each other. Alternatively, the pose from an earlier time or another time is used to assist in the pose determination for a later or different time. For example, the pose from an earlier time is used to initialize the comparison to later determine the pose. The minimization (e.g., minimization using RANSAC) may have fewer iterations where the initial pose is closer to the actual pose. Collecting video of the tracked object helps to refine the pose estimate of the target object. For example, after the feature extraction and matching (template matching of pose determination) are performed for the first frame, the generated information is cached for use for one or more subsequent frames. Based on the frame rate of the camera, the target object may have a small amount of movement between different frames. Utilizing a pose estimate from one or more previous frames provides the matching algorithm with an initial guess for the pose, which the algorithm then refines into the new estimate. Time-based pose estimation and refinement helps to maintain a stable pose estimate throughout the duration of the tracking and accelerates the processing time for generating the pose estimate.

The acts may be repeated or performed for one or more objects, such as any objects from the set of expected objects and corresponding templates. The pose is determined for only one object (e.g., ultrasound transducer) or for each of multiple objects. Any number of occluding objects may be modeled for dealing with occlusion.

Figure 4 shows one embodiment of an ultrasound system. The ultrasound system is part of an ultrasound scanner or is a computer separate from the ultrasound scanner. In other implementations, the system is for other medical imaging (not ultrasound), a therapy system, or a surgical procedure system. A computer without a scanner or as part of a scanner is provided for determining pose of a medical device.

The ultrasound system includes an image processor 400, memory 410, and display 420. The ultrasound system also includes the camera 300 for sensing (imaging) the medical device (e.g., the transducer 200) and/or the transducer 200 for scanning the patient 430. The display 420, image processor 400, and memory 410 may be part of a medical imaging or therapy system or may be a computer, server, workstation, or another system communicatively connected with the medical imaging or therapy system for image processing.

Additional, different, or fewer components may be provided. For example, a computer network is included for remote image processing and/or display. As another example, one or more machine-learned models are stored in the memory 410 and applied by the processor 400 to detect, segment, extract, and/or determine pose. In yet another example, a beamformer, scan converter, and/or one or more filters are provided for ultrasound imaging.

The camera 300 is a single camera configured to image the acoustic transducer 200. Other cameras for other purposes may be in the room, but the camera 300 is the only camera used for pose determination.

The camera 300 is a depth sensor, optical camera, 3D camera, infrared camera, thermal camera, and/or another type of camera. LIDAR, 2.5D, color depth (RGBD), or another depth camera may be used. The camera 300 may include a separate processor for determining depth measurements from images and/or detecting objects represented in images, or the image processor 400 determines the depth measurements and/or detects objects from images captured by the camera 300. The camera 300 may directly measure depth from the camera 300 to the patient. The depth may be relative to the camera 300 and/or a bed or table 616. Alternatively, a camera without depth sensing is used. A light projector may be provided.

The camera 300 is directed to the patient 430 and/or acoustic transducer 200. The camera 300 may be part of or connected to the ultrasound scanner. In one embodiment, the camera 300 is positioned on a boom, robotic arm, ceiling, and/or walls. The field of view of the camera 300 as positioned includes the region of the patient 430 where the transducer 200 is to be used.

The camera 300 is calibrated to the transducer 200, patient 430, bed 440, ultrasound system, or another coordinate system. The known relationship of the camera 300 to another device or room and the known camera parameters allow a pose determined relative to the camera to be transformed to pose relative to the other device or room.

The acoustic transducer 200 is a probe for scanning. The acoustic transducer 200 is the part of the scanner that scans the patient. For example, a beamformer uses the acoustic transducer 200 to scan the patient 430 with ultrasound for therapy and/or imaging. Other medical devices may be used instead, such as for interacting with the patient in other ways than imaging or scanning.

The image processor 400 is a control processor (e.g., controller), general processor, digital signal processor, three-dimensional data processor, graphics processing unit, application specific integrated circuit, field programmable gate array, artificial intelligence processor, digital circuit, analog circuit, combinations thereof, or another now known or later developed device for image processing to determine pose. The image processor 400 is a single device, a plurality of devices, or a network. For more than one device, parallel or sequential division of processing may be used. Different devices making up the image processor 400 may perform different functions, such as detecting objects in an image by one device and determining pose by another device. In one embodiment, the image processor 400 is a control processor or other processor of a medical scanner. The image processor 400 operates pursuant to and is configured by stored instructions, hardware, and/or firmware to perform various acts described herein.

In one implementation, the image processor 400 is configured to determine a pose of the acoustic transducer 200 from an image from the single camera 300. The acoustic transducer 200 as represented in an image from the camera 300 is detected, such as detecting by application of a machine-learned classifier. The pose is determined based on a template of the transducer. By matching the detected transducer 200 in the image to the template at different orientations, scales, and/or positions, the pose of the detected transducer is determined as the orientation, scale, and/or position of the best match (e.g., minimum difference).

In one approach, the image processor 400 is configured to extract features of the acoustic transducer from the image or segmentation. A machine-learned model, template (e.g., statistical shape model), or another image process is applied to identify the locations of semantic and/or generalized features of the acoustic transducer 200 in the image. The image processor 400 determines the pose based on the extracted features relative to template features.

The template used may be specific to the acoustic transducer 200 (e.g., template for same model of transducer). Alternatively, the template is of a generalized transducer that may apply to different models of transducer. The generalized transducer of the template is not specific to the acoustic transducer 200 captured in the image. The image processor 400, using extracted features, is configured to determine the pose of the acoustic transducer 400 using the generalized template.

The display 420 is a CRT, LCD, projector, plasma, printer, tablet, smart phone, or another now known or later developed display device for displaying the captured image, a pose, a medical image, a pre-operative image with a graphical representation of the acoustic transducer (or other medical device) posed according to the determined pose. The display 420 may display scan information, such as a medical image or treatment course.

The camera data (images), segmentation, extracted features (landmarks), machine-learned model(s), template(s), rendered ultrasound images, and/or other information are stored in a non-transitory computer readable memory, such as the memory 410. The memory 410 is an external storage device, RAM, ROM, database, and/or a local memory (e.g., solid state drive or hard drive). The same or different non-transitory computer readable media may be used for the instructions and other data. The memory 410 may be implemented using a database management system (DBMS) and residing on a memory, such as a hard disk, RAM, or removable media. Alternatively, the memory 410 is internal to the processor 400 (e.g., cache).

The instructions for implementing the methods, processes, and/or techniques discussed herein are provided on non-transitory computer-readable storage media or memories, such as a cache, buffer, RAM, removable media, hard drive, or other computer readable storage media (e.g., the memory 410). Computer readable storage media include various types of volatile and nonvolatile storage media. The functions, acts or tasks illustrated in the figures or described herein are executed in response to one or more sets of instructions stored in or on computer readable storage media. The functions, acts or tasks are independent of the particular type of instructions set, storage media, processor or processing strategy and may be performed by software, hardware, integrated circuits, firmware, micro code, and the like, operating alone or in combination.

In one embodiment, the instructions are stored on a removable media device for reading by local or remote systems. In other embodiments, the instructions are stored in a remote location for transfer through a computer network. In yet other embodiments, the instructions are stored within a given computer, CPU, GPU, or system. Because some of the constituent system components and method steps depicted in the accompanying figures may be implemented in software, the actual connections between the system components (or the process steps) may differ depending upon the manner in which the present embodiments are programmed.

Listed below are various Illustrative Embodiments. The Illustrative Embodiments summarize different combinations of aspects. Other combinations of any of the aspects with any other one or more of the aspects may be provided. Aspects from one type (e.g., method or system) may be used in another type (system or method).

Illustrative Embodiment 1. A method for pose estimation of an ultrasound transducer, the method comprising: capturing, with a single camera, an image of the ultrasound transducer, the ultrasound transducer being held and occluded by a user in the image; detecting the ultrasound transducer in the image; determining a pose of the ultrasound transducer as detected in the image from the single camera; and ultrasound imaging using the ultrasound transducer with scan data aligned by the determined pose.

Illustrative Embodiment 2. The method of Illustrative Embodiment 1, wherein capturing comprises capturing with a color depth camera, the image comprising a color point cloud, and wherein detecting comprises detecting, at least in part, based on the color point cloud.

Illustrative Embodiment 3. The method of any of Illustrative Embodiments 1-2, wherein capturing comprises capturing the image of the ultrasound transducer where the ultrasound transducer is free of an added optical marker.

Illustrative Embodiment 4. The method of any of Illustrative Embodiments 1-3, wherein detecting comprises segmenting the ultrasound transducer in the image.

Illustrative Embodiment 5. The method of any of Illustrative Embodiments 1-4, further comprising extracting a plurality of features of the detected ultrasound transducer in the image.

Illustrative Embodiment 6. The method of Illustrative Embodiment 5, wherein extracting comprise extracting the features as semantic features and general cues.

Illustrative Embodiment 7. The method of any of Illustrative Embodiments 1-6, wherein determining the pose comprises comparing the ultrasound transducer as detected to an ultrasound transducer template.

Illustrative Embodiment 8. The method of Illustrative Embodiment 7, wherein comparing comprises comparing the ultrasound transducer as detected to the ultrasound transducer template, the ultrasound transducer template representing a generalized transducer that is not specific to the ultrasound transducer being detected.

Illustrative Embodiment 9. The method of any of Illustrative Embodiments 7-8, wherein comparing comprises testing different orientations of the ultrasound transducer template to the ultrasound transducer as detected, the testing resulting in the pose where a difference in the testing is minimized.

Illustrative Embodiment 10. The method of any of Illustrative Embodiments 7-9, wherein comparing comprises comparing feature vectors output by an encoder in response to input of the ultrasound transducer as detected and the ultrasound transducer template.

Illustrative Embodiment 11. The method of any of Illustrative Embodiments 7-10, further comprising extracting a plurality of features of the detected ultrasound transducer in the image; wherein the ultrasound transducer template has more template features than a number of the plurality; and wherein comparing comprises comparing with an outlier rejection.

Illustrative Embodiment 12. The method of any of Illustrative Embodiments 1-11, wherein capturing, detecting, and determining are performed repetitively over time, and wherein the pose from an earlier time is used to initialize the determining of the pose at a later time.

Illustrative Embodiment 13. The method of any of Illustrative Embodiments 1-12, further comprising modeling a hand of the user, and wherein detecting the ultrasound transducer and/or determining the pose accounts for the modeling of the hand.

Illustrative Embodiment 14. The method of any of Illustrative Embodiments 1-13, wherein ultrasound imaging comprises rendering from a three-dimensional representation with data of the three-dimensional representation aligned in the three-dimensional representation based on the pose.

Illustrative Embodiment 15. An ultrasound system comprising: a single camera configured to image an acoustic transducer occluded in part by a user; and an image processor configured to determine a pose of the acoustic transducer from an image from the single camera, the determination being based on a template of the transducer.

Illustrative Embodiment 16. The ultrasound system of Illustrative Embodiment 15, wherein the single camera comprises a color depth sensor.

Illustrative Embodiment 17. The ultrasound system of any of Illustrative Embodiments 15-16, wherein the image processor is configured to extract features of the acoustic transducer from the image and determine the pose based on the extracted features relative to template features.

Illustrative Embodiment 18. The ultrasound system of any of Illustrative Embodiments 15-17, wherein the template comprises a representation of a generalized transducer that is not specific to the acoustic transducer of the image.

Illustrative Embodiment 19. A method for pose estimation of a medical device, the method comprising: capturing, with a single camera, an image of the medical device during use of the medical device on or in a patient, the medical device being held and occluded by a user in the image; detecting the medical device in the image and a part of the user in the image; and determining a pose of the medical device as detected in the image from the single camera, the pose determined utilizing the detected part of the user in the image.

Illustrative Embodiment 20. The method of Illustrative Embodiment 19, wherein determining the pose comprises: removing features detected for the medical device based on the part of the user; using an orientation of the part of the user to indicate the pose; and/or solving for the pose considering both the medical device in the image and the part of the user in the image.

Various improvements described herein may be used together or separately. Although illustrative embodiments of the present invention have been described herein with reference to the accompanying drawings, it is to be understood that the invention is not limited to those precise embodiments, and that various other changes and modifications may be affected therein by one skilled in the art without departing from the scope of the invention which is defined by the claims.

## Claims

1. A method for pose estimation of an ultrasound transducer, the method comprising:
capturing, with a single camera, an image of the ultrasound transducer, the ultrasound transducer being held and occluded by a user in the image;
detecting the ultrasound transducer in the image;
determining a pose of the ultrasound transducer as detected in the image from the single camera; and
ultrasound imaging using the ultrasound transducer with scan data aligned by the determined pose.

2. The method of claim 1, wherein capturing comprises:
capturing with a color depth camera, the image comprising a color point cloud, and wherein detecting comprises detecting, at least in part, based on the color point cloud; and/or
capturing the image of the ultrasound transducer where the ultrasound transducer is free of an added optical marker.

3. The method of claim 1 or 2, wherein detecting comprises segmenting the ultrasound transducer in the image.

4. The method of any one of claims 1 - 3, further comprising extracting a plurality of features of the detected ultrasound transducer in the image.

5. The method of claim 4, wherein extracting comprises extracting the features as semantic features and general cues.

6. The method of any one of claims 1 - 5, wherein determining the pose comprises comparing the ultrasound transducer as detected to an ultrasound transducer template.

7. The method of claim 6, wherein comparing comprises at least one of:
comparing the ultrasound transducer as detected to the ultrasound transducer template, the ultrasound transducer template representing a generalized transducer that is not specific to the ultrasound transducer being detected;
testing different orientations of the ultrasound transducer template to the ultrasound transducer as detected, the testing resulting in the pose where a difference in the testing is minimized; and/or
comparing feature vectors output by an encoder in response to input of the ultrasound transducer as detected and the ultrasound transducer template.

8. The method of claim 6 or 7, further comprising extracting a plurality of features of the detected ultrasound transducer in the image;
wherein the ultrasound transducer template has more template features than a number of the plurality; and
wherein comparing comprises comparing with an outlier rejection.

9. The method of any one of claims 1 - 8, wherein capturing, detecting, and determining are performed repetitively over time, and wherein the pose from an earlier time is used to initialize the determining of the pose at a later time.

10. The method of any one of claims 1 - 9, further comprising modeling a hand of the user, and wherein detecting the ultrasound transducer and/or determining the pose accounts for the modeling of the hand.

11. The method of any one of claims 1 - 10, wherein ultrasound imaging comprises rendering from a three-dimensional representation with data of the three-dimensional representation aligned in the three-dimensional representation based on the pose.

12. An ultrasound system comprising:
a single camera configured to image an acoustic transducer occluded in part by a user; and
an image processor configured to determine a pose of the acoustic transducer from an image from the single camera, the determination being based on a template of the transducer, in particular, according to a method of any one of claims 6 - 8.

13. The ultrasound system of claim 12, wherein the single camera comprises a color depth sensor.

14. The ultrasound system of claim 12 or 13, wherein the image processor is configured to extract features of the acoustic transducer from the image and determine the pose based on the extracted features relative to template features.

15. The ultrasound system of any one of claims 12 - 14, wherein the template comprises a representation of a generalized transducer that is not specific to the acoustic transducer of the image.

16. A method for pose estimation of a medical device, the method comprising:
capturing, with a single camera, an image of the medical device during use of the medical device on or in a patient, the medical device being held and occluded by a user in the image;
detecting the medical device in the image and a part of the user in the image; and
determining a pose of the medical device as detected in the image from the single camera, the pose determined utilizing the detected part of the user in the image.

17. The method of claim 16, wherein determining the pose comprises:
removing features detected for the medical device based on the part of the user;
using an orientation of the part of the user to indicate the pose; and/or
solving for the pose considering both the medical device in the image and the part of the user in the image.
